# EUROPEAN PATENT SPECIFICATION

(11) **EP 1 377 247 B1**
(45) Date of publication and mention of the grant of the patent: **21.06.2006**
(21) Application number: 02744905.7
(22) Date of filing: 27.02.2002
(51) Int. Cl.: A61F 15/00

(54) **TAMPON PACK**
TAMPONPACKUNG
ENSEMBLE DE TAMPON

(30) Priority: 28.02.2001 DE 10109608
(43) Date of publication of application: 07.01.2004
(73) Proprietor: JOHNSON & JOHNSON GmbH, 40474 Düsseldorf (DE)
(72) Inventor: LOCHTE, Karin, 42103 Wuppertal (DE); MEYBOOM, Astrid, 42781 Haan (DE); KERNER, Werner, 42277 Wuppertal (DE)
(74) Representative: Metten, Karl-Heinz
(86) International application number: PCT/EP2002/002112
(87) International publication number: WO 2002/067837

(56) References cited:
- WO-A-99/00096
- DE-U- 7 401 694
- US-A- 4 170 305

## Description

The invention relates to a primary tampon pack with a tampon, for feminine hygiene or for medical purposes, which has a cylindrical absorbent core, at one end of which a retrieval string is provided, and which is enclosed in a form-fitting manner by packaging which can be opened by means of a tear-open strip which divides the packaging circumferentially into two packaging sleeves, wherein the tear-open strip has a width which is at least 25 % of the length of the cylindrical absorbent core.

Such a primary tampon pack is disclosed in US-A-4 170 305. The tear-open strip is defined by the perforation lines which are provided to tear the packaging of the rapped catamenial tampon and free the tampon. The film packaging in this case encloses the absorbent core together with the retrieval string in an essentially airtight manner. The tampon is wrapped in the film, an exposed border region being formed in the axial direction. If the user then uses a finger to grip behind a tear-open tab formed by the end of the tear-open strip and the packaging film, a tear-open strip can be drawn off centrally from the tampon. The packaging sleeves which enclose the two ends of the tampon can then be slipped off one after the other by the user.

In a primary tampon pack in which the cylindrical absorbent core has a length of approximately 50 mm, the width of the tear-open strip is approximately 8 mm, with the result that, once said tear-open strip has been removed, approximately 80% of the length of the tampon still remains protected.

DE 197 53 665 C2 describes a tampon in which the surface treatment of a wholly or partially thermoplastic, nonwoven covering material by calendering under the action of heat and pressure causes the tampon to be smoothed, with the result that, even before and after the days in which menstruation is relatively heavy or if only light vaginal discharges occur, the tampon can be inserted into the vagina, and also removed again, more easily and conveniently.

WO 99/0096 [sic] discloses tampons in which the absorbent core is provided with a perforated plastic film which at least partially encloses the core. This plastic film has a relatively smooth surface, with the result that it is also the case here that the tampon is inserted, for example into the vagina, noticeably more conveniently.

It has been found, then, that it is no longer possible for the packaging which has been used up until now for the primary tampon pack to be used, in particular, for this tampon provided with the plastic film and for the tampon having the thermoplastic covering material. The packaging sleeves at the ends of the tampon are difficult to draw off. The problem is also increased if the tampon in its individual pack was exposed to increased levels of humidity and/or elevated temperature over a relatively long period of time. Under such conditions, the packaging is relatively difficult to remove even in the case of a tampon made of untreated nonwoven materials.

The object of the invention is to provide an improved primary tampon pack, the packaging of which is easy to release and, in the process, gives the same protection as has been the case up until now.

This object is achieved by a primary tampon pack as claimed in claim 1. Advantageous configurations form the subject matter of the subclaims.

The invention provides for the packaging to be configured that the packaging is provided with a roughness-imparting layer at least in the region which encloses the end located opposite the retrieval string, in order for the removal of the packaging sleeve provided here to be facilitated further. A similar layer may, if desired, also be provided at the end having the retrieval string.

It is also preferred if the tear-open strip is offset from the center of the tampon in the direction of the end having the retrieval string, for example if it is offset by at least 5% of the overall length of the tampon, more preferably by at least 10% of the overall length of the tampon. The amount by which the tear-open strip is offset is expediently coordinated here with the width of the tear-open strip. Only a relatively small packaging sleeve then remains at that end of the tampon which has the retrieval string, and, despite the forces acting between the plastic film and the packaging material, said packaging sleeve does not require any significant force to be exerted in order to be drawn off. The packaging sleeve at the front, opposite end can be slipped off relatively easily on account of the conicity of the tampon.

The width of the tear-open strip is preferably between 25% and 70%, more preferably between 45% and 55%, of the length of the cylindrical absorbent core.

Provision may also be made for the packaging to be coated with an anti-friction agent on the surface directed toward the tampon.

The packaging preferably consists of polypropylene film or cellulose film.

The packaging in a primary tampon pack according to the invention is particularly suitable for the tampon in which the absorbent core is provided with a perforated plastic film which at least partially encloses the core and extends up to, or into the vicinity of, the end provided with the retrieval string, but also for a tampon in which the absorbent core is provided with a friction-reducing coating.

In order to make it possible for the tear-open strip to be torn in a controlled manner, two spaced-apart incisions may be provided on the exposed edge of the packaging, said edge extending in the axial direction of the tampon, and form a tear-open aid for the tear-open strip.

A tear-open tab is preferably formed between the incisions.

The invention will be described in more detail hereinbelow with reference to the attached drawings, in which:
- Figure 1: shows a side view of a tampon with a perforated plastic film;
- Figure 2: shows a side view of a primary tampon pack; and
- Figure 3: shows diagrams (a) to (d) which show the changes in weight and the force for removing the packaging sleeves in the case of a tampon according to figure 1, comparisons being made between a conventional pack and a pack described herein;
- Figure 4: shows a schematic illustration of the exposed edge of the packaging with incisions as a tear-open aid;
- Figure 5: shows a schematic illustration of the exposed edge of the packaging, a tear-open tab being formed on the tear-open strip; and
- Figure 6: shows a modification of the tear-open strip with tear-open tab in an illustration similar to figure 5.

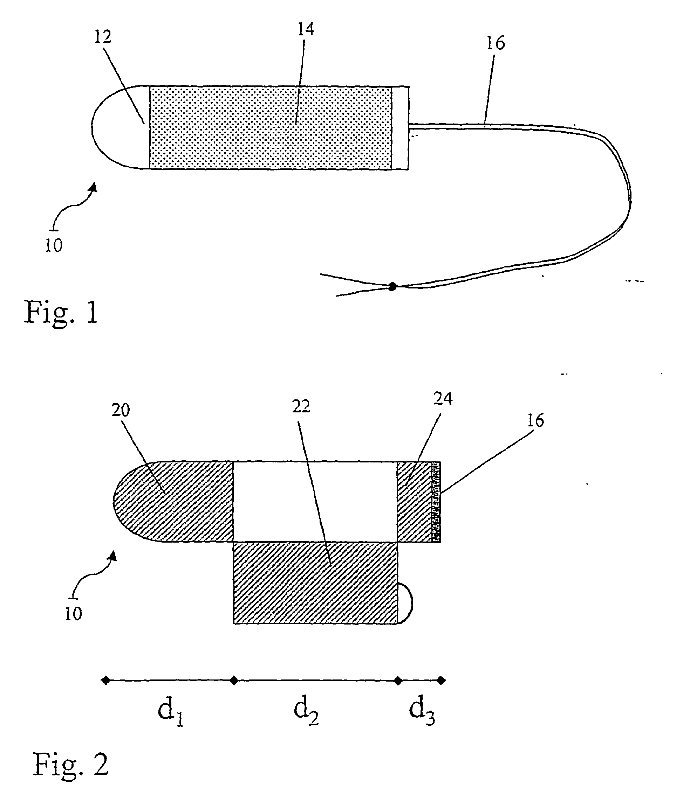

Figure 1 shows a side view of a tampon as is known, for example, from WO 99/00096. An essentially cylindrical absorbent core 12 made of fluid-absorbing material is provided, this core having a retrieval string 16 at one end. The absorbent core 12 is provided with a perforated plastic film 14 virtually over its entire length. The plastic film 14 has a relatively smooth surface, and the holes serve for transporting fluid into the interior of the tampon, where the fluid is taken up by the absorbent core 12. Instead of the plastic film 4, it is also possible for coating to have been carried out, as is described in DE 197 53 665 C2. The invention is not restricted to use with tampons of this type; it may also be used with tampons which, at least in the surface region, consist of merely untreated nonwoven materials.

Figure 2 shows a tampon 10 in a pack, a tear-open strip 22 having been drawn off from the tampon 10. The tip of the tampon is still protected in a packaging sleeve 20, and the retrieval string 16 is wound up and located, together with that end of the tampon 10 assigned to it, in a packaging sleeve 24. The tear-open strip 22 is offset from the center of the tampon 10, to be precise in the direction of the end provided with the retrieval string 16. If the length of the tampon 10 corresponds to d1 + d2 + d3, where d1 is the length of the packaging sleeve 20, d2 is the width of the tear-open strip 22 and d3 is the length of the packaging sleeve 24, then, in the embodiment illustrated in figure 2, d1 > d3. Furthermore, the width d2 of the tear-open strip 22 is approximately 50% of the length of the tampon 10. In a specific embodiment, d1 = 16 mm, d2 = 24 mm and d3 = 10 mm.

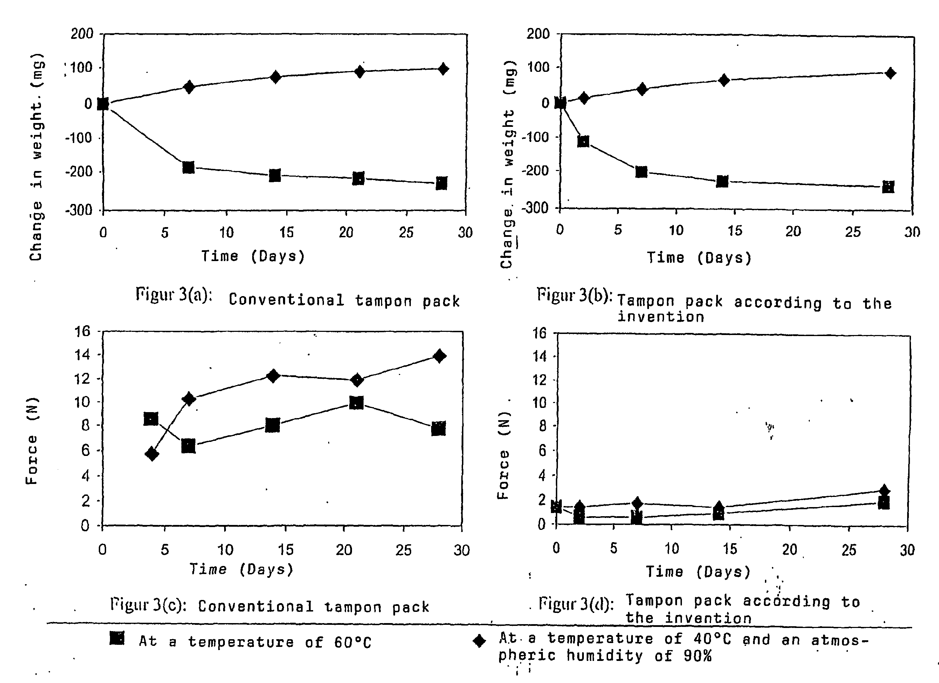

Figures 3(a) and 3(b) show, in a comparison, the change in weight over time of a primary tampon pack according to the present invention if the primary tampon pack is exposed to an elevated temperature of 60°C and to an elevated temperature of 40°C at 90% air humidity over 30 days.

Figure 3(a) shows that, with merely an elevation in temperature, the weight of the conventional primary tampon pack decreases but, with an elevation in temperature and simultaneously high air humidity, a considerable increase may be observed. The same behavior is shown in figure 3(b) for the primary tampon pack according to the invention. It is thus the case that the protective function of the packaging is not lost in the case of a primary tampon pack according to the present invention.

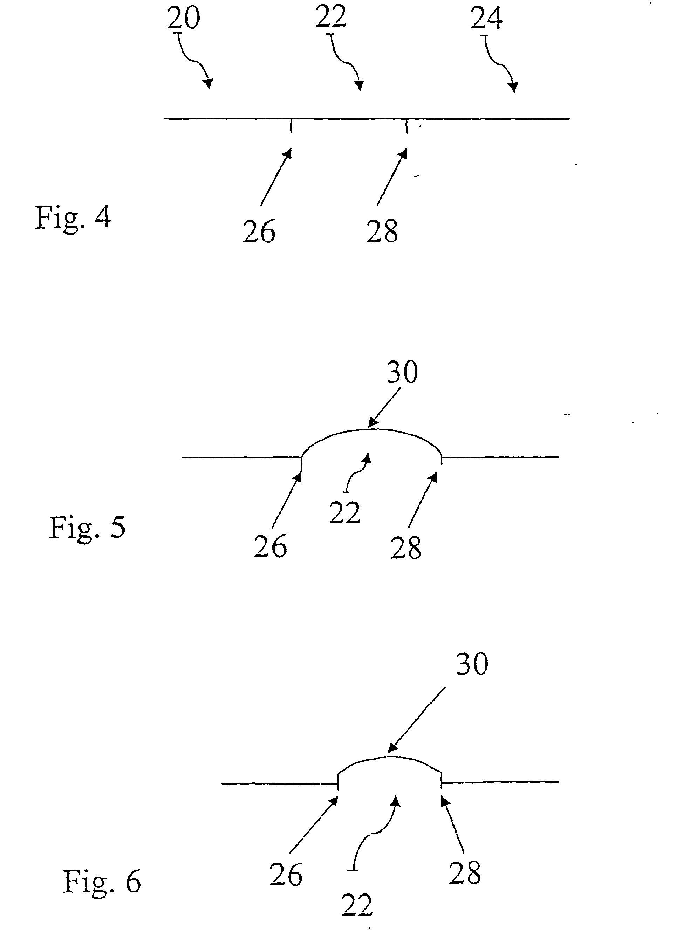

Figures 3(c) and 3(d) show, for the conventional primary tampon pack and for the primary tampon pack described herein, the level of force which has to be exerted at respectively selected points in time in order to remove the tear-open strip and the packaging sleeves. The conventional primary tampon pack, in comparison with the primary tampon pack described herein, requires forces which are higher by a factor of 3 to approximately 7 than the forces in the case of the primary tampon pack according to the present invention, diagram 3(d), in particular, showing clearly that these forces remain approximately at the same low value even over a long period of time of 30 days. Figure 4 shows a schematic illustration of the exposed tampon-packaging edge, relatively short incisions 26, 28, which, in the practical embodiment, are approximately 1-2 mm in length, extending from the edge. These incisions 26, 28 serve as a tear-open aid for the tear-open strip 22, with the result that the latter can be separated from the packaging sleeves 20, 24 in a controlled and largely defined manner.

Figure 5 shows a configuration in which there is provided on the tear-open strip 22 a tear-open tab 30, of which the contour is curved in arcuate form and opens out into the exposed edge of the packaging at the incisions 26, 28. This tear-open tab 30 helps the user in raising the tear-open strip 22 off from the packaging.

A modification of the tear-open tab 30 is shown in figure 6. Rather than the arcuate contour opening out into the exposed edge in this case, the tear-open tab 30 [lacuna] guided out of the packaging in extension of the incisions 26, 28, this making it easier to grip behind the tear-open tab 30 and/or the tear-open strip 22.

The features of the invention which are disclosed in the above description, in the drawing and in the claims may be essential both individually and in any desired combination for the purpose of realizing the invention.

## Claims

1. A primary tampon pack with a tampon for feminine hygiene or for medical purposes, which has a cylindrical absorbent core (12), at one end of which a retrieval string is provided, and which is enclosed in a form-fitting manner by packaging which can be opened by means of a tear-open strip which divides the packaging (20, 22, 24) circumferentially into two packaging sleeves (20, 24), wherein the tear-open strip (22) has a width (d2) which is at least 25% of the length (d1 + d2 +d3) of the cylindrical absorbent core (12),
**characterized in that** said packaging (20, 22, 24) is provided with a roughness-imparting layer at least in the region in (20) which encloses the end located opposite the retrieval string (16).

2. The primary tampon pack as claimed in claim 1, wherein the packaging (20, 22, 24) is coated with an anti-friction agent on the surface directed toward the tampon (10).

3. The primary tampon pack as claimed in claim 1, wherein the tear-open strip (22) is offset from the center of the tampon (10) in the direction of the end having the retrieval string (16).

4. The primary tampon pack as claimed in claim 1, wherein the width (d2) of the tear-open strip (22) is between 25% and 70%, preferably between 45% and 55%, of the length of the cylindrical absorbent core (12).

5. The primary tampon pack as claimed in claim 1, wherein the packaging (20, 22, 24) consists of polypropylene film or cellulose film.

6. The primary tampon pack as claimed in one of claims 1 to 5, wherein the absorbent core (12) is provided with a perforated plastic film (14) which at least partially encloses the core and extends up to, or into the vicinity of, the end provided with the retrieval string (16).

7. The primary tampon pack as claimed in one of claims 1 to 5, wherein the absorbent core (12) is provided with a friction-reducing coating.

8. The primary tampon pack as claimed in one of claims 1 to 7, wherein two spaced aparat incisions (26, 28) are provided on the exposed edge of the packaging (20, 22, 24), said edge extending in the axial direction of the tampon (10), and form a tear-open aid for the tear-open strip.

9. The primary tampon pack as claimed in one of claims 1 to 8, wherein a tear-open tab (30) is formed between the incisions (26, 28).

## Patentansprüche

1. Primäre Tamponpackung mit einem Tampon für Frauenhygiene oder für medizinische Zwecke, der einen zylindrischen absorbierenden Kern (12) umfaßt, welcher an einem Ende ein Rückholbändchen aufweist, und der in formgerechter Weise von einer Verpackung umschlossen ist, die mit Hilfe eines Aufreißfadens geöffnet werden kann, der die Verpackung (20, 22, 24) umfänglich in zwei Verpackungshüllen (20, 24) aufteilt, wobei der Aufreißfaden (22) eine Breite (d2) aufweist, die zumindest 25 % der Länge (d1 + d2 + d3) des zylindrischen absorbierenden Kerns (12) entspricht,
**dadurch gekennzeichnet, daß** besagte Verpackung (20, 22, 24) zumindest in dem Bereich in (20), welcher das Ende umschließt, das sich gegenüber dem Rückholbändchen (16) befindet, mit einer Schicht versehen ist, die ihr Rauhigkeit verleiht.

2. Die primäre Tamponpackung, wie in Anspruch 1 beansprucht, **dadurch gekennzeichnet, daß** die Verpackung (20, 22, 24) mit einem Antifriktionsmittel auf der zum Tampon (10) gerichteten Oberfläche beschichtet ist.

3. Die primäre Tamponpackung, wie in Anspruch 1 beansprucht, **dadurch gekennzeichnet, daß** der Aufreißfaden (22) gegen die Mitte des Tampons (10) in Richtung auf das Ende, das mit dem Rückholbändchen (16) versehen ist, versetzt ist.

4. Die primäre Tamponpackung, wie in Anspruch 1 beansprucht, **dadurch gekennzeichnet, daß** die Breite (d2) des Aufreißfadens (22) zwischen 25 % und 70 %, vorzugsweise zwischen 45 % und 55 % der Länge des zylindrischen absorbierenden Kerns (12) ausmacht.

5. Die primäre Tamponpackung, wie in Anspruch 1 beansprucht, **dadurch gekennzeichnet, daß** die Verpackung (20, 22, 24) aus einer dünnen Polypropylen- oder Zellstoffschicht besteht.

6. Die primäre Tamponpackung, wie in einem der Ansprüche 1 bis 5 beansprucht, **dadurch gekennzeichnet, daß** der absorbierende Kern (12) eine perforierte dünne Kunststoffschicht (14) aufweist, die zumindest zum Teil den Kern umschließt und sich bis zum Ende oder bis in die Nähe des Endes erstreckt, das mit dem Rückholbändchen (16) versehen ist.

7. Die primäre Tamponpackung, wie in einem der Ansprüche 1 bis 5 beansprucht, **dadurch gekennzeichnet, daß** der absorbierende Kern (12) eine reibungsverringernde Beschichtung aufweist.

8. Die primäre Tamponpackung, wie in einem der Ansprüche 1 bis 7 beansprucht, **dadurch gekennzeichnet, daß** zwei beabstandete Einschnitte (26, 28) am freiliegenden Rand der Verpackung (20, 22, 24) vorhanden sind, wobei besagter Rand sich in axialer Richtung des Tampons (10) erstreckt und eine Aufreißhilfe für den Aufreißfaden ausbildet.

9. Die primäre Tamponpackung, wie in einem der Ansprüche 1 bis 8 beansprucht, **dadurch gekennzeichnet, daß** eine Aufreißlasche (30) zwischen den Einschnitten (26, 28) ausgebildet ist.

## Revendications

1. Conditionnement primaire de tampon avec un tampon pour l'hygiène féminine ou destiné à des fins médicales, qui possède un coeur absorbant cylindrique (12), à une extrémité duquel un cordon de retrait est prévu, et qui est enveloppé de manière s'ajustant à la forme par un emballage qui peut être ouvert au moyen d'une bande d'ouverture déchirable qui divise l'emballage (20, 22, 24) de manière circonférentielle en deux manchons d'emballage (20, 24), dans lequel la bande d'ouverture déchirable (22) possède une largeur (d2) qui est au moins 25 % de la longueur (d1 + d2 + d3) du coeur absorbant cylindrique (12),
**caractérisé en ce que** ledit emballage (20, 22, 24) est muni d'une couche procurant de la rugosité au moins dans la zone (20) qui enveloppe l'extrémité située face au cordon de retrait (16).

2. Conditionnement primaire de tampon selon la revendication 1, dans lequel l'emballage (20, 22, 24) est revêtu d'un agent anti-frottement sur la surface tournée vers le tampon (10).

3. Conditionnement primaire de tampon selon la revendication 1, dans lequel la bande d'ouverture déchirable (22) est décalée par rapport au centre du tampon (10) dans la direction de l'extrémité possédant le cordon de retrait (16).

4. Conditionnement primaire de tampon selon la revendication 1, dans lequel la largeur (d2) de la bande d'ouverture déchirable (22) mesure entre 25 % et 70 %, de préférence entre 45 % et 55 %, de la longueur du coeur absorbant cylindrique (12).

5. Conditionnement primaire de tampon selon la revendication 1, dans lequel l'emballage (20, 22, 24) est constitué d'un film de polypropylène ou de cellophane.

6. Conditionnement primaire de tampon selon l'une quelconque des revendications 1 à 5, dans lequel le coeur absorbant (12) est muni d'un film plastique perforé (14) qui enveloppe au moins partiellement le coeur et s'étend jusqu'à, ou à proximité de, l'extrémité munie du cordon de retrait (16).

7. Conditionnement primaire de tampon selon l'une quelconque des revendications 1 à 5, dans lequel le coeur absorbant (712) est muni d'un revêtement réduisant le frottement.

8. Conditionnement primaire de tampon selon l'une quelconque des revendications 1 à 7, dans lequel deux incisions espacées l'une de l'autre (26, 28) sont prévues sur le bord exposé de l'emballage (20, 22, 24), ledit bord s'étendant dans la direction axiale du tampon (10), et forment une aide à l'ouverture déchirable pour la bande d'ouverture déchirable.

9. Conditionnement primaire de tampon selon l'une quelconque des revendications 1 à 8, dans lequel une languette d'ouverture déchirable (30) est formée entre les incisions (26, 28).
